# EUROPEAN PATENT SPECIFICATION

(11) **EP 2 872 179 B1**
(45) Date of publication and mention of the grant of the patent: **11.01.2017**
(21) Application number: 13734433.9
(22) Date of filing: 09.07.2013
(51) Int. Cl.: A61K 47/26, A61K 9/19

(54) **A METHOD TO PRODUCE A MEDICINAL PRODUCT COMPRISING A BIOLOGICALLY ACTIVE PROTEIN AND THE RESULTING PRODUCT**
VERFAHREN ZUR HERSTELLUNG EINES MEDIZINISCHEN PRODUKTS MIT EINEM BIOLOGISCH AKTIVEN PROTEIN UND RESULTIERENDES PRODUKT
PROCÉDÉ POUR PRODUIRE UN PRODUIT MÉDICINAL COMPRENANT UNE PROTÉINE BIOLOGIQUEMENT ACTIVE ET PRODUIT AINSI OBTENU

(30) Priority: 10.07.2012 US 201261669797 P
(43) Date of publication of application: 20.05.2015
(73) Proprietor: Intervet International B.V., 5831 AN Boxmeer (NL)
(72) Inventor: O'CONNELL, Kevin, Elkhorn, Nebraska 68022 (US); BUCHANAN, Sandhya, Florham Park, New Jersey 07932 (US)
(74) Representative: Intervet International B.V.
(86) International application number: PCT/EP2013/064422
(87) International publication number: WO 2014/009328

(56) References cited:
- WO-A1-2009/092703
- WO-A1-2010/125084
- WO-A1-2010/125087
- WO-A2-2011/072218
- US-A1- 2004 154 317
- US-A1- 2007 190 163

## Description

### Field of the invention

The present invention is in the field of preservation of biologically active proteins, in particular for storage purposes. In particular the present invention pertains to a method for producing a medicinal product comprising a biologically active protein, the method comprising preservation of the protein by freeze-drying in a protective matrix comprising a sugar. The invention also pertains to the resulting product, which in particular is a freeze-dried body comprising the protein, stabilised by the sugar.

### Background art

Biologically active proteins (also denoted simply "proteins" in this specification) are generally unstable when stored in solid media or liquid solutions. In particular the secondary and tertiary structure of the proteins, which depend on hydrogen bonds within the molecule, are prone to degeneration. Since the secondary and tertiary structure in particular lead to the recognisable domains of the protein structure, preservation of this structure is often key for preservation of the biologic activity of the protein. Whole organisms are often surrounded by a complex lipid based membrane and integral proteins which are structurally stabilized by the nature of the surrounding aqueous solution of which the ideal properties of the structure include a low free energy state. That low free energy state is difficult to obtain and shifting structure as well as proteases produced by the organism itself or accompanying host cells, make it very difficult to stabilize structures in a liquid form. Lyophilisation (freeze-drying) processes, wherein aqueous compositions, usually comprising water as the main solvent, are frozen and then dried by sublimation, are commonly used to stabilise biologically active proteins. Removal of the solvent and substitution by a matrix comprising protective molecules such as sugar molecules, may increase the stability of the protein by preventing degradation and denaturation of this protein.

In lyophilisation, the protein is commonly mixed as a suspension in water with a protective agent, frozen and the dehydrated by sublimation and secondary drying. The low temperatures of freezing and drying by sublimation, together with the low surface to volume ratios involved, can require long drying periods. Such long drying periods may result in structural damage to the protein. Increasing the drying temperature to reduce drying times is often not an option since the drying temperature has to remain significantly below the glass-transition temperature of the protective matrix. Moreover, high drying temperatures inherently lead to loss of the biologic activity of the protein. A particular problem comes about when using a non-polymeric sugar as a protective agent. As commonly known, non-polymeric sugars are very suitable for stabilising biologically active proteins, but they have the inherent disadvantage that the glass transition temperature of the resulting matrix is relatively low (typically below 100°C, often between 20 and 45°C). In particular when high amounts of sugar are being used, and the frozen body has a considerable thickness (typically above 2 mm), this leads to very long drying times in the range of 72 - 96 hours. In particular when the amount of non-polymeric sugar comes close to 20% w/w, the resulting matrix becomes so dense that drying times increase exponentially (therefore, in the art up to 15-16% (w/w) maximally is applied when aiming at homogenously dried bodies). Long drying times inherently lead to a significant loss in biologic activity of the protein and are very unattractive from a manufacturing point of view.

In the art, a solution has been provided in US 5,565,318. This reference proposes to use a polymeric sugar (such as dextran or ficoll) as protective agent. Since the glass transition temperature of such a polymeric sugar is generally higher than these of non-polymeric sugars (typically above 200°C vs below 100°C), one can use substantially higher drying temperatures to arrive at shorter drying times. However, the stabilising properties of such polymeric sugars are far less superior than these of non-polymeric sugars. Also, higher drying temperatures to obtain shorter drying times may also lead to a higher rate of degradation and or denaturation of the biologically active protein during the freeze-drying process itself.

In US 2010/0297231 an alternative solution is provided wherein a formulation comprising the biologically active protein and a polyol (which may be a non-polymeric sugar such as fructose, mannose, maltose, lactose, glucose, trehalose, ribose, rhamnose, sorbitol, xylitol etc.) is expanded into a foam, whereafter the foam is frozen and dried into a stable dry foam composition. Since the foam comprises a thickness of 2 mm or less, and the surface to volume ratio is extremely high, short drying times can be obtained even with high percentages of non-polymeric sugars. A disadvantage of the known method is that the foam takes up a lot of space in the freeze dryer for the relative low amount of active material that is being processed and also, a foam needs to be ground into a powder for further use, such as administration by inhalation, or for obtaining predetermined quantities of dried material for reconstitution in a liquid to enable use in every day practice. A further disadvantage is that typically a foaming agent is comprised in the formulation. This limits the use of the resulting product.

Therefore, preservation of biologically active proteins, in particular to arrive at an easy to use end-product allowing a broad spectrum of applications and having a matrix that provides high preservation properties still pose a significant challenge. It is an object of the invention to meet that challenge.

### Summary of the invention

In order to meet the object of the invention a method is provided for producing a medicinal product comprising a biologically active protein comprising the steps of providing an aqueous composition comprising a solvent, the biologically active protein and between 20% w/w and 60% w/w of a non-polymeric sugar, freezing the composition, thereby forming at least one frozen body comprising the solvent in frozen form, putting the frozen body in a drying apparatus while being carried by a support, the support comprising one or more restraining elements that define one or more boundaries of the support, wherein at most 30% of the surface of the body is contiguous with the one or more restraining elements, reducing the pressure in the drying apparatus below atmospheric pressure, and providing heat to the body (typically via conduction and/or radiation) in order to sublimate the frozen solvent of the body and obtain a dried body.

To inventor's surprise by using a freeze-drying technique that is based on obtaining individual frozen bodies starting from a composition comprising a high amount of a non-polymeric sugar, which bodies are dried making sure that the greater part of their surface is not contiguous to a restraining element (such as a closed bottom or wall) of a container, one can dry the bodies in a relatively short time, substantially below 72-96 hours despite the fact that the surface to volume ratio is far below the ratio when applying a foam, and despite the fact that the thickness may even be above 2 mm. It is currently believed that the relatively high amount of surface that is not contiguous with an element that would prevent the free flow of sublimated solvent out of the frozen body (such as for example the glass bottom and wall of typical freeze-drying vials), is essential in obtaining the advantageous drying results of these high-sugar compositions. Recognising this, it is clear that the invention does not essentially depend on the form of the body. For example when using cubes, pyramid's, bricks, stars, diamonds, tic-tac shaped bodies or any other form, when placed on a support such that at most 30% of the surface of the body is contiguous with the one or more restraining elements of the support, sufficient free surface of each body is available for the sublimated solvent to leave the body.

Indeed, lyophilisation techniques wherein frozen bodies are dried while the greater part of their surface is not contiguous to a restraining element of a container are known in the prior art, e.g. WO 2010/125087. However, this reference does not disclose the lyophilisation of a composition comprising a biologically active protein starting from a solution comprising at least 20% w/w of a non-polymeric sugar. For compounds other than biologically active proteins, in particular for stable (small) molecules, fast drying times can be obtained by increasing the amount of heat needed for sublimation of the frozen solvent, since denaturation is often not a problem. For biologically active proteins however this is not an option. Therefore typically, when drying a body constituted from a frozen aqueous solvent comprising a biologically active protein, a solvent with 3-10% w/w of a non-polymeric sugar is being used.

The invention is also embodied in a medicinal product in the form of a substantial homogenous freeze-dried body having a volume between 50 and 1000 µl comprising a biologically active protein, the protein being dispersed in a solid matrix of a non-polymeric sugar, characterised in that the body comprises between about 21 and 72 % w/v of the said sugar. The 21-72% w/v in the ultimate dried body corresponds to about 20-60% w/w of the sugar in the starting aqueous composition (which can be calculated using the formula for calculating the approximate density of a solution of sugar in water: density ≈ 0.3723 times (sugar concentration in grams/litre) + 1002.2). This body can be individually handled, needs no grinding or other form of re-working before being actually applied as a medicinal product, and is highly suitable for preserving the biologically active protein given the high density of the non-polymeric sugar in the matrix. Preferably, the body is a spheroid.

### Definitions

*To produce:* to provide for further use, for example for use in a laboratory, for use in a plant, as an intermediate product or for use by a consumer (end-user). To produce includes to manufacture, i.e. to produce in a controlled repeatable manner to provide lager amounts of the same product.

*Medicinal product*: any product that can be used to prevent, treat or cure a disease or disorder of a living organism, in particular a human being or non-human animal.

*A biologically active protein:* any protein that has a three-dimensional structure such that the protein is able to induce or interfere with a physiological process in a living organism, such as a metabolic process or an immunological process. The term biologically active protein may denote a full-length protein or a fragment thereof. Examples of biologically active proteins are enzymes, toxins, toxoids, any (other) immunological protein (for example a surface protein of a micro-organism or a fragment thereof), an antibody or a fragment thereof etc. Vaccines typically contain one or more biologically active proteins, either as part of a living or killed micro-organism, or as an isolated or recombinantly expressed subunit of such a micro-organism.

*A solvent*: any carrier fluid, the fluid being suitable for dissolving and/or dispersing the substance to be carried.
% *w*/*w*: percentage mass over total mass of the composition.
*% w*/*v*: percentage mass over total volume of the composition.

*A sugar*: any of a group of water-soluble carbohydrates of relatively low molecular weight and typically having a sweet taste. The term sugar includes reducing sugars (such as fructose and maltose), non-reducing sugars (such as sucrose and trehalose), sugar alcohols (such as xylitol and sorbitol) and sugar acids (such as gluconic acid and tartaric acid).

*A non-polymeric sugar*: mono-, di-, tri-, and oligomeric sugar molecules, comprising at most six monomeric sugar molecules.

*A body.* having a volume such that it can be handled individually by hand (manually), typically having a volume equal to or above 50µl (which equals a sphere having a radius of about 2 mm). Preferably it is small enough to be used as a tablet for administration to an animal (the term "animal" including a human being), in particular having a volume below 2000µl. Preferably the volume of the body is between 50µl and 1500µl, typically below 1000µl, in particular between 75µl and 750µl, further in particular between 100µl and 500µl.

*Dry*: less than 5% of residual moisture, in particular less than 4½, 4, 3½, 3, 2½, 2%, 1½%, or 1% residual moisture.

*A restraining element*: actual element that constitutes a restraining surface that acts in defining the boundaries of a supporting element. Typically a restraining element is a closed (continues) bottom, side-wall or lid of a container. It may however also be the wiring of a supporting wire-rack or grid-wall rack.
*Contiguous:* to share a boundary.
*A spheroid:* A body that is shaped like a sphere but is not necessarily perfectly round. It may for example be an oblate body, a prolate body, a body that partly consists of a sphere and partly of an oblate or prolate body, a sphere with one or more dents, rims or other irregularities, combinations of the above etc.

### Embodiments of the invention

In an embodiment the frozen solvent is sublimated in less than 48 hours. The heat flow is preferably chosen such that the body is dried within 48 hours. This significantly lowers the time the biologically active protein is subjected to the relatively high drying temperatures. Surprisingly, in the current set up, such short drying times can be obtained without losing significant activity of the protein. Preferably, the frozen solvent is sublimated in less than 36 hours, or even in 16 to 24 hours. Compared with prior art lyophilisation methods for drying bodies comprising a biologically active protein and at least 20% w/w of a non-polymeric sugar, this is a very short drying time.

In another embodiment at most 20% of the surface of the body is contiguous with the one or more restraining elements of the support, preferably even at most 10%. It appears that by having more surface available for the free sublimation of solvent (by having less surface of the body being contiguous with a restraining element of the support), the drying time can be further decreased while preserving the biologic activity of the protein.

Preferably the frozen body is a spheroid such that the percentage of the surface of the body that is contiguous with a restraining element of the support is typically below 3%, or even near 0%. In a further embodiment the spheroid has a volume between 50µl and 1000µl which approximates a radius of the spheroid between 2 and 6 mm (depending on the shape of the spheroid).

In an embodiment wherein the restraining element of the support is a floor, the body being provided lying on this floor, and the support is open to allow radiation to freely pass to the said floor, at least a part of the heat is provided by emitting heat radiation from a radiation source present in the drying apparatus above the support, to reach the frozen body. It has appeared that adequate drying of the high sugar containing bodies can be achieved when providing the heat (at least partly) via radiation. Radiation has the advantage that it does not depend on a heat conductive material to transfer the heat.

In another embodiment at least a part of the heat is provided by conduction of heat via the restraining element that is contiguous with the frozen body, in line with common lyophilisation processes. Despite the fact that in the current method at most 30% of the surface of the body us contiguous with a restraining element of the support (up to even a figure as low is 1-3%), whereas in prior art methods this is typically above 50%, it appears that this is sufficient to provide an adequate heat flow to the body.

In yet another embodiment wherein the restraining element of the support is a floor, the body being provided lying on this floor, and the support is open to allow radiation to freely pass to the said floor, the heat is provided by emitting heat radiation from a radiation source present in the drying apparatus above the support to reach the body in combination with conduction of heat via the restraining element that is contiguous with the frozen body (a set-up which is known from EP 2249810). A combined heat flow by conduction and radiation has proven to be very adequate for drying bodies that have a relatively low percentage of their body in contact with a support.

In an embodiment, the amount of the sugar in the aqueous composition is chosen from the group that consists of the ranges 20-55% w/w, 20-50% w/w, 20-45% w/w, 25-45% w/w, 25-40% w/w, 25-35% w/w and 27-30% w/w. Preferably, the amount of sugar is higher than 25% w/w, typically around 27% w/w.

In an embodiment the sugar comprises monomeric and/or dimeric molecules, in particular chosen from the group consisting of glucose, galactose, maltose, sucrose, trehalose, fructose, lactose, saccharose, mannitol, sorbitol and xylitol.

In an embodiment wherein the restraining element of the support is a floor, multiple frozen bodies are positioned in the form of a monolayer on the said floor while being dried. This way, large quantities of frozen bodies can be dried in an efficient way.

In another embodiment the residual moisture content in the dried body is less than 2% w/w, typically between 0.5 and 2% w/w, for example between 1 and 2% w/w.

The invention will be explained in more detail using the following examples and figures.
Figure 1 schematically shows a first type of support, viz. a glass vial, filled with frozen spheroids or a liquid composition, frozen after being filled in the support.
Figure 2 schematically shows a drying set-up in a lyophilising apparatus.
Figure 3 schematically shows an alternative drying set-up in a lyophilising apparatus.
Figure 4 schematically shows a further alternative drying set-up in a lyophilising apparatus.

Example 1 is a first example of freeze-drying high sugar compositions.
Example 2 is a second example of freeze-drying high sugar compositions.
Example 3 is a third example of freeze-drying high sugar compositions.
Example 4 is a fourth example of freeze-drying high sugar compositions.
Example 5 is a fifth example of freeze-drying high sugar compositions.
Example 6 provides stability data for medicinal products according to the invention.

### Figure 1

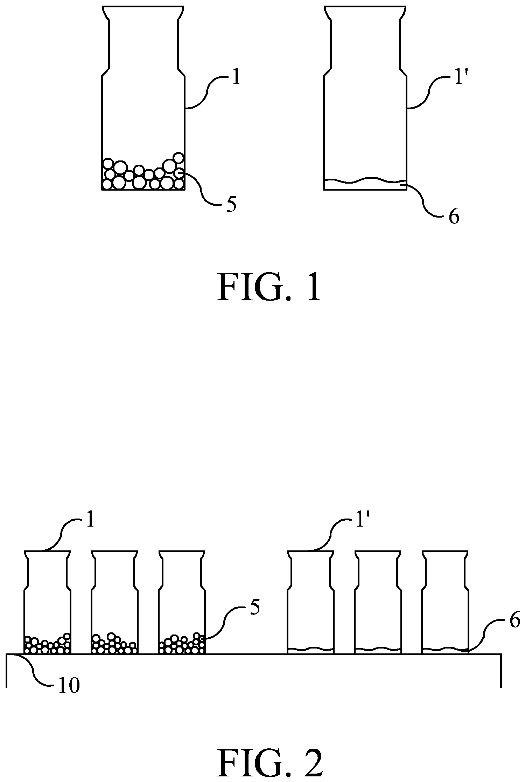

Figure 1 schematically shows a first type of support, viz. a glass vial, filled with frozen spheroids or a liquid composition, frozen after being filled in the support. The figure depicts a first glass vial (1) which is filled with about 15 frozen spheres (5) of about 110µl each. The figure also depicts a second glass vial (1') filled with 1.5 ml of liquid composition that has been frozen to from one frozen cake (6).

### Figure 2

Figure 2 schematically shows a drying set-up in a lyophilising apparatus (apparatus as a whole not shown). The figure depicts a shelf (10) which carries vials 1 and 1'. The vials 1 are filled with frozen spheres 5 and the vials 1' are filled with a cake of frozen liquid (6). The shelf can be heated via built-in electrical heaters (not shown).

### Figure 3

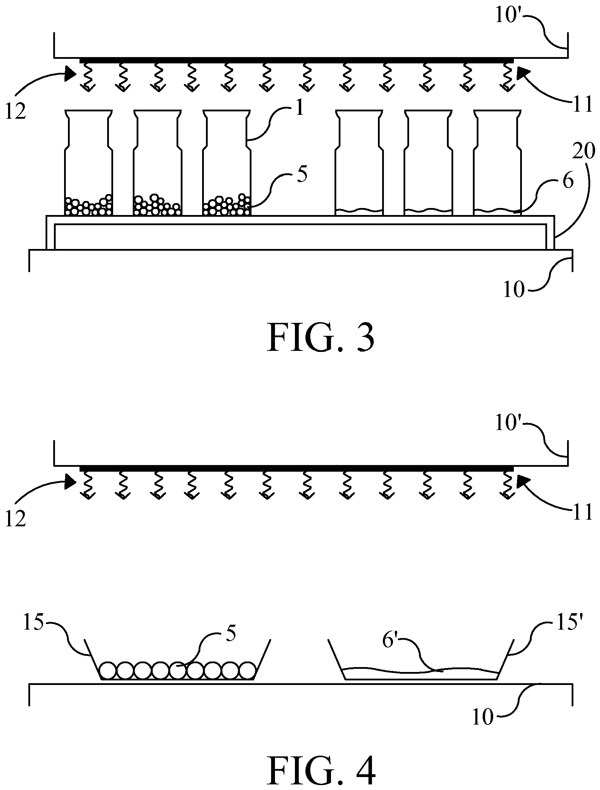

Figure 3 schematically shows an alternative drying set-up in a lyophilising apparatus. In this set up the vials are put on a heightened support (20) which is virtually thermally insulated from the heated shelf (10). This way, the vials will receive virtually no heat via conduction of heat generated in the shelf (10). Above the vials there is a second heated shelf (10'), which shelf is provided with a black coating (11). This black coating provides that the shelf will act as a good radiating source, emitting heat radiation 12 (mainly infrared light) towards the vials.

### Figure 4

Figure 4 schematically shows a further alternative drying set-up in a lyophilising apparatus. This set-up uses two shelves, one heated shelf 10 which acts as a first heat source and one heated shelf 10' which acts as a second heat source (via black coating 11 that provides a radiating surface for the bottom of shelf 10', corresponding to the set-up as depicted in figure 3). Standing on the first shelf 10 are open containers 15 and 15'. Container 15 is filled with a monolayer of frozen spheres (5), whereas the second container 15' is filled with a frozen liquid (6').

Examples 1 to 5 are proof-of-principle examples showing that using the method according to the invention, a substantially homogenous dried body can be obtained within a reasonable fast drying cycle, despite the fact that over 20% w/w of a non-polymeric sugar is present in the initial aqueous composition. For these examples a simple solution of 30% sucrose (w/v) in water was used (which approximates 27% w/w). Part of this aqueous composition was used to make frozen spheres having a volume of approximately 100µl. For this a process as described in WO 2010/125084 was used, in particular the process as mentioned on line 33, page 20, to line 2, page 21 (in conjunction with figures 1, 2, 3 and 4 of the same international application). The frozen spheres are used in the examples as described below.

In example 1, a first set of three 10 ml glass lyophilising vials (1) having a diameter of approximately 20 mm, are filled with about 15 spheres (5), equating about 1.5 ml of the initial aqueous composition. In the vials, this results in about 1½ layer of spheres. In this set-up, less then 10% of the surface of the frozen spheres (about 1-3%) is contiguous with the one or more walls (restraining elements) of the supporting vial. A second set of three of corresponding vials (1') is filled with 1.5 ml of the liquid composition as described here above, whereafter the liquid is frozen. This way, about 60% of the surface of the frozen body is contiguous with the one or more walls of the supporting vial. The resulting filled vials are schematically shown in Figure 1.

These vials are put on a shelf in a standard lyophilising apparatus, the prime parts of which are schematically shown in figure 2, which has beforehand been brought to a temperature of about -45°C. This lyophiliser is subjected to the following freeze-drying cycle (Table 1).

**Table 1**

| Phase | | | | | | |
|---|---|---|---|---|---|---|
| Freezing | Temp [°C] | -45 | -45 | | | |
| | Time [m] | 0 | 1 | | | |
| | Vacuum [mbar] | NA | NA | | | |
| Initial Sublimation | Temp [°C] | -45 | | | | |
| | Time [m] | 0 | | | | |
| | Vacuum [mbar] | 0.04 | | | | |
| Sublimation | Temp [°C] | -45 | -45 | 35 | 35 | 35 |
| | Time [m] | 0 | 10 | 123 | 960 | 240 |
| | Vacuum [mbar] | 0.04 | 0.04 | 0.04 | 0.04 | 0.34 |

As can be seen in Table 1, after loading the shelf with the filled containers the shelf (10) is firstly kept at a temperature of -45°C for 1 minute (the "Freezing" phase). Herewith the frozen bodies are brought to a temperature of -45°C. The pressure is kept atmospheric. Then, the pressure is lowered to 0.04 mbar while the temperature of the shelf is kept at minus 45°C ("Initial sublimation"). Under these conditions, the frozen liquid already sublimates and heat is supplied to the pellets via conduction through the shelf. However, the speed of sublimation under these conditions is relatively low. To increase the speed of sublimation, the shelf is brought to a temperature of 35°C ("Sublimation"), and kept at that temperature for about 22 hours (total of 1333 minutes, as indicated under "Sublimation"). The pressure is kept at the low value of 0.04 mbar. After that, the sublimation process is completed and up to about 98% of the frozen liquid has left the frozen bodies, thereby transforming into dried bodies. Then, dried nitrogen gas with a temperature of about 20°C is led into the lyophiliser until the pressure is about atmospheric. This takes about 2 minutes. Then the door can be opened to take out the vials.

Within the drying time of about 22 hours, the spheres were in general dry and in good shape, with some insignificant incomplete drying at some spots. In the second set of vials however, the frozen body had turned into a mess of foam and syrupy liquid.

In example 2 the same lyophilising apparatus as used in example 1 is used, albeit that the bottom of the shelf above the shelf carrying the vials is provided with a black PTFE (polytetrafluoroethylene) plate. By intimate contact between this black plate and the shelf, this plate is warmed to virtually the same temperature as the shelf itself and hence will act as a radiation heat source. Still, only a small portion of the radiation will reach the frozen bodies directly since the glass wall of the vial will absorb and reflect most radiation. A comparable set-up is described in WO 2010/125084 in conjunction with Figure 5 of that reference. By having a heat flow to the vials not only by conduction via the vials standing on a shelf, but also by radiation from the shelf above, the drying performance can be improved. Still, only for the spheres this leads to an improved drying result, namely that the spheres can be dried in about 20 hours. The liquid filled vials however still lead to a very poor inhomogenous drying result.

In example 3 the lyophilising set-up as described in conjunction with example 2 is used, albeit that a heat flow via conduction is virtually ruled out by putting the vials on a heightened support, keeping them virtually insulated from the shelf by which they are carried. This set-up is shown in Figure 3. By choosing this set-up, the required heat flow towards the frozen bodies is virtually completely obtained via radiation from the shelf above. The drying cycle, which now includes the actual freezing of the material from a liquid into a solid frozen body, is shown beneath in Table 2.

**Table 2**

| Phase | | | | | | | |
|---|---|---|---|---|---|---|---|
| Freezing | Temp [°C] | -45 | -45 | -20 | -20 | -45 | -45 |
| | Time [m] | 0 | 10 | 15 | 60 | 15 | 20 |
| | Vacuum [mbar] | NA | NA | NA | NA | NA | NA |
| Initial Sublimation | Temp [°C] | -45 | | | | | |
| | Time [m] | 0 | | | | | |
| | Vacuum [mbar] | 0.04 | | | | | |
| Sublimation part 1 | Temp [°C] | -45 | -45 | 35 | 35 | | |
| | Time [m] | 0 | 10 | 123 | 960 | | |
| | Vacuum [mbar] | 0.04 | 0.04 | 0.04 | 0.04 | | |
| Sublimation part 2 | Temp [°C] | 35 | | | | | |
| | Time [m] | 240 | | | | | |
| | Vacuum [mbar] | 0.34 | | | | | |

This gave the same drying result as described in conjunction with example 1, in about the same drying time.

In the next example, example 4, it is tried to obtain a reasonably good drying result of the body as present in the second set of vials as described in example 1. These vials were put on a shelf of the same lyophilising apparatus, thus only using a heat flow via conduction through the shelf. A drying cycle according to Table 3 was used, which led to a long drying time of over 76 hours.

Although a markedly improved drying result was obtained when compared with the result of example 1, the resulting dried body still had substantial melted regions, deep cracks and some foaming. This means that even longer drying times would be needed to achieve a drying result which is comparable with that obtainable using the method according to the invention.

**Table 3**

| Phase | | | | | | | |
|---|---|---|---|---|---|---|---|
| Freezing | Temp [°C] | -45 | -45 | | | | |
| | Time [m] | 0 | 30 | | | | |
| | Vacuum [mbar] | NA | NA | | | | |
| Initial Sublimation | Temp [°C] | -45 | | | | | |
| | Time [m] | 0 | | | | | |
| | Vacuum [mbar] | 0.04 | | | | | |
| Sublimation 1 | Temp [°C] | -25 | -25 | 20 | 20 | 35 | 35 |
| | Time [m] | 15 | 2880 | 600 | 360 | 240 | 240 |
| | Vacuum[mbar] | 0.04 | 0.04 | 0.04 | 0.04 | 0.04 | 0.20 |
| Sublimation 2 | Temp [°C] | 35 | | | | | |
| | Time [m] | 200 | | | | | |
| | Vacuum[mbar] | 0.27 | | | | | |

In example 5, another type of support is used for drying the frozen bodies (see figure 4). This support is an open container (15, 15') having a width of about 20 cm, a length of about 30 cm and a height of about 4 cm. The container is made of a heat conducting plastic material, in this case carbon black filled polyethyleneterephtalate. The container can be put in a heat conducting contact with a shelf upon which they rest. The difference with such a container when comparing it with a standard freeze-drying vial is that such a container is in essence open, and thus allows radiation to freely pass to the floor of said container to reach the frozen material. In the arrangement shown in figure 4, one container 15 is filled with a monolayer of frozen spheres 5 and the other container 15' is filled with a frozen layer 6' of the sucrose containing composition (with a thickness of about 5½ mm). The drying set-up is the set up as described in conjunction with example 2. By heating the shelves, the frozen bodies may receive heat via the heated bottom and side walls of the containers and by irradiation from the heated shelf above. A drying cycle as indicated in Table 4 is used fro drying the frozen material.

The result of this drying cycle is that the individual spheres are nicely dried, being of good shape and have virtually no incomplete drying spots. The layer of frozen sucrose composition however is inhomogeneously dried, with substantial incomplete drying and foaming.

**Table 4**

| Phase | | | | | | | |
|---|---|---|---|---|---|---|---|
| Freezing | Temp [°C] | -45 | -45 | -20 | -20 | -45 | -45 |
| | Time [m] | 0 | 10 | 15 | 60 | 15 | 20 |
| | Vacuum [mbar] | NA | NA | NA | NA | NA | NA |
| Initial Sublimation | Temp [°C] | -45 | | | | | |
| | Time [m] | 0 | | | | | |
| | Vacuum [mbar] | 0.04 | | | | | |
| Sublimation 1 | Temp [°C] | -45 | -45 | 35 | 35 | | |
| | Time [m] | 0 | 10 | 123 | 960 | | |
| | Vacuum [mbar] | 0.04 | 0.04 | 0.04 | 0.04 | | |
| Sublimation 2 | Temp [°C] | 35 | | | | | |
| | Time [m] | 240 | | | | | |
| | Vacuum [mbar] | 0.34 | | | | | |

Example 6 provides some embodiments of medicinal products comprising a biologically active protein that are obtained using a method according to the invention, in particular a method as described here above in conjunction with example 2. The first product (denoted as "CDV") is vaccine in the form of lyospheres that can serve to formulate a vaccine for injection by re-suspension of one or more spheres in water-for-injection. Each sphere, having a volume of approximately 100µl, contains live attenuated canine distemper virus at a titre of about 7 (Iog10 of the TCID50). This product is obtained in two forms, both at pH 7.2 using a 10 mM KPO₄ buffer, a first form according to the prior art having 3.7% (w/w) of sucrose as a stabilising agent (and in addition, 0.8% w/v gelatin and 1.0% w/v NZ amine as bulking agents), and a second form having 21.4% (w/w) of non-polymeric sugar (15.3% (w/w) sucrose and 6.1% (w/w) trehalose) as a stabilising agent (next to 5.2% w/v arginine, 0.8% w/v gelatin and 1.0% w/v NZ amine as bulking agents).These spheres were subjected to a test wherein the spheres were stored at 45°C for up to 4 weeks. The resulting titre was determined after 1, 2 and 4 weeks of storage. The results are indicated in table 5.

**Table 5Titres of CDV spheres after storage at 45°C**

| storage time in weeks | CDV Sphere 3.7% (w/w) sugar | CDV Sphere 21.4% (w/w) sugar |
|---|---|---|
| | log 10 TCID50 | log 10 TCID50 |
| 0 | 7.5 | 7.2 |
| 1 | 4.6 | 4.7 |
| 2 | 4.3 | 5.1 |
| 3 (estimate, interpolation) | 3.4 | 5.0 |
| 4 | 2.6 | 5.0 |

The second product (denoted as "CPI") is also a vaccine in the form of lyospheres that can serve to formulate a vaccine for injection by re-suspension of one or more spheres in water-for-injection. Each sphere, having a volume of approximately 100µl, contains live attenuated canine parainfluenza virus at a titre of about 7 (Iog10 of the TCID50). This product is obtained in two forms, a first form according to the prior art having 3.7% (w/w) of sucrose as a stabilising agent and a second form having 21.4% (w/w) of non-polymeric sugar as a stabilising agent (both as indicated here-above). These spheres were subjected to a test wherein the spheres were stored at 45°C for up to 4 weeks. The resulting titre was determined after 1, 2 and 4 weeks of storage. The results are indicated in table 6.

**Table 6 Titres of CPI spheres after storage at 45°C**

| storage time in weeks | CPI Sphere 3.7% (w/w) sugar | CPI Sphere 21.4% (w/w) sugar |
|---|---|---|
| | log 10 TCID50 | log 10 TCID50 |
| 0 | 6.7 | 6.9 |
| 1 | 4.6 | 5.0 |
| 2 | 4.2 | 5.3 |
| 3 (estimate, interpolation) | 3.3 | 5.1 |
| 4 | 2.5 | 4.9 |

The third product (denoted as "CAV2") is also a vaccine in the form of lyospheres that can serve to formulate a vaccine for injection by re-suspension of one or more spheres in water-for-injection. Each sphere, having a volume of approximately 100µl, contains live attenuated canine adeno virus type 2 at a titre of about 5 (Iog10 of the TCID50). This product is obtained in two forms, a first form according to the prior art having 3.7% (w/w) of sucrose as a stabilising agent, and a second form having 22.8% (w/w) of sucrose as a stabilising agent. These spheres were subjected to a test wherein the spheres were stored at 45°C for up to 4 weeks. The resulting titre was determined after 1,2,3 and 4 weeks of storage. The results are indicated in table 7.

**Table 7 Titres of CAV2 spheres after storage at 45°C**

| storage time in weeks | CAV2 Sphere 3.7% (w/w) sugar | CAV2 Sphere 22.8% (w/w) sugar |
|---|---|---|
| | log 10 TCID50 | log 10 TCID50 |
| 0 | 5.0 | 4.7 |
| 1 | 2.6 | 4.3 |
| 2 | 2.8 | 3.9 |
| 3 (estimate interpolation) | 2.7 | 4.1 |
| 4 | 2.5 | 4.2 |

The above experiments with the CPi, CAV2 and CDV antigens were repeated with a different non-polymeric sugar mixture above 20% w/w, to see whether comparable results could be obtained. In this experiment the stabiliser contained 21.6% (w/w) of non-polymeric sugar (6.1% (w/w) sucrose and 15.5% (w/w) trehalose) in a 10 mM KPO4 buffer at pH 7.2. The bulking agent was the same as in the other experiment (5.2% w/v arginine, 0.8% w/v gelatin and 1.0% w/v NZ amine). The spheres were again subjected to a test wherein the spheres were stored at 45°C for up to 4 weeks. The resulting titre was determined after 1, 2 and 4 weeks of storage. The results are shown in table 8. An equivalent result as the previous formulation could be obtained with this stabiliser.

**Table 8**

| storage time in weeks | CPi Sphere 21.6% (w/w) sugar | CAV2 Sphere 21.6% (w/w) sugar | CDV Sphere 21.6% (w/w) sugar |
|---|---|---|---|
| | log 10 TCID50 | log 10 TCID50 | log 10 TCID50 |
| 0 | 6.50 | 4.42 | 7.08 |
| 1 | 5.50 | 4.33 | 5.83 |
| 2 | 5.67 | 4.33 | 5.42 |
| 4 | 4.67 | 4.33 | 5.33 |

## Claims

1. A method for producing a medicinal product comprising a biologically active protein comprising the steps of:
- providing an aqueous composition comprising a solvent, the biologically active protein and between 20% w/w and 60% w/w of a non-polymeric sugar,
- freezing the composition, thereby forming at least one frozen body comprising the solvent in frozen form,
- putting the frozen body in a drying apparatus while being carried by a support, the support comprising one or more restraining elements that define one or more boundaries of the support, wherein at most 30% of the surface of the body is contiguous with the one or more restraining elements,
- reducing the pressure in the drying apparatus below atmospheric pressure,
- providing heat to the body in order to sublimate the frozen solvent of the body and obtain a dried body, the freeze-dried body having a volume between 50 and 1000 µl.

2. A method according to claim 1, **characterised in that** the frozen solvent is sublimated in less than 48 hours.

3. A method according to claim 2, **characterised in that** the frozen solvent is sublimated in less than 36 hours.

4. A method according to claim 3, **characterised in that** the frozen solvent is sublimated in 16 to 24 hours.

5. A method according to any of the preceding claims, **characterised in that** at most 20% of the surface of the body is contiguous with the one or more restraining elements of the support.

6. A method according to claim 5, **characterised in that** at most 10% of the surface of the body is contiguous with the one or more restraining elements of the support.

7. A method according to any of the preceding claims, **characterised in that** the frozen body is a spheroid.

8. A method according to any of the preceding claims, wherein the restraining element of the support is a floor, the body being provided lying on this floor, and the support is open to allow radiation to freely pass to the said floor, **characterised in that** at least a part of the heat is provided by emitting heat radiation from a radiation source present in the drying apparatus above the support, to reach the frozen body.

9. A method according to any of the claims 1 to 7, wherein the restraining element of the support is a floor, the body being provided lying on this floor, and the support is open to allow radiation to freely pass to the said floor, **characterised in that** the heat is provided by emitting heat radiation from a radiation source present in the drying apparatus above the support to reach the body in combination with conduction of heat via the restraining element that is contiguous with the frozen body.

10. A method according to any of the preceding claims, **characterised in that** the amount of the sugar in the aqueous composition is chosen from the group that consists of the ranges 20-55% w/w, 20-50% w/w, 20-45% w/w, 25-45% w/w, 25-40% w/w, 25-35% w/w and 27-30% w/w.

11. A method according to any of the preceding claims, **characterised in that** the sugar comprises monomeric and/or dimeric molecules.

12. A method according to claim 11, **characterised in that** the sugar comprises glucose, galactose, maltose, sucrose, trehalose, fructose, lactose, saccharose, mannitol, sorbitol and/or xylitol.

13. A method according to any of the preceding claims, **characterised in that** a residual moisture content in the dried body is less than 2% w/w.

14. A medicinal product in the form of a substantial homogenous freeze-dried body having a volume between 50 and 1000 µl comprising a biologically active protein, the protein being dispersed in a solid matrix of a non-polymeric sugar, **characterised in that** the body comprises between about 21 and 72 % w/v of the said sugar.

15. A product in the form of a substantial homogenous freeze-dried body having a volume between 50 and 1000 µl, comprising a biologically active protein, the body being obtainable via a method according to any of the claims 1 to 13.

## Patentansprüche

1. Verfahren zum Herstellen eines Arzneimittels, das ein biologisch aktives Protein umfasst, welches die Schritte umfasst:
- Bereitstellen einer wässrigen Zusammensetzung, die ein Lösungsmittel, das biologisch aktive Protein und zwischen 20 % w/w und 60 % w/w eines nichtpolymeren Zuckers umfasst,
- Einfrieren der Zusammensetzung, wodurch mindestens ein eingefrorener Körper gebildet wird, der das Lösungsmittel in eingefrorener Form umfasst,
- Legen des eingefrorenen Körpers in einen Trockenapparat, während er von einer Auflage getragen wird, wobei die Auflage ein oder mehrere Zurückhalteelemente umfasst, die eine oder mehrere Grenzen der Auflage definieren, wobei höchstens 30 % der Fläche des Körpers an das eine oder die mehreren Zurückhalteelemente angrenzt,
- Reduzieren des Drucks im Trockenapparat auf unter Atmospährendruck,
- Bereitstellen von Wärme an den Körper, um das eingefrorene Lösungsmittel des Körpers zu sublimieren und einen getrockneten Körper zu erhalten, wobei der gefriergetrocknete Körper ein Volumen zwischen 50 und 1000 µl aufweist.

2. Verfahren nach Anspruch 1, **dadurch gekennzeichnet, dass** das eingefrorene Lösungsmittel in weniger als 48 Stunden sublimiert wird.

3. Verfahren nach Anspruch 2, **dadurch gekennzeichnet, dass** das eingefrorene Lösungsmittel in weniger als 36 Stunden sublimiert wird.

4. Verfahren nach Anspruch 3, **dadurch gekennzeichnet, dass** das eingefrorene Lösungsmittel in 16 bis 24 Stunden sublimiert wird.

5. Verfahren nach einem der vorstehenden Ansprüche, **dadurch gekennzeichnet, dass** höchstens 20 % der Fläche des Körpers an das eine oder die mehreren Zurückhalteelemente der Auflage angrenzt.

6. Verfahren nach Anspruch 5, **dadurch gekennzeichnet, dass** höchstens 10 % der Fläche des Körpers an das eine oder die mehreren Zurückhalteelemente der Auflage angrenzt.

7. Verfahren nach einem der vorstehenden Ansprüche, **dadurch gekennzeichnet, dass** der eingefrorene Körper ein Sphäroid ist.

8. Verfahren nach einem der vorstehenden Ansprüche, wobei das Zurückhalteelement der Auflage ein Boden ist und der Körper auf diesem Boden liegend bereitgestellt wird und die Auflage offen ist, um zu ermöglichen, dass Strahlung ungehindert zu dem Boden passieren kann, **dadurch gekennzeichnet, dass** mindestens ein Teil der Wärme durch Emittieren von Wärmestrahlung von einer Strahlungsquelle bereitgestellt wird, die in der Trockenapparat über der Auflage vorhanden ist, um den eingefrorenen Körper zu erreichen.

9. Verfahren nach einem der Ansprüche 1 bis 7, wobei das Zurückhalteelement der Auflage ein Boden ist und der Körper auf diesem Boden liegend bereitgestellt wird und die Auflage offen ist, um zu ermöglichen, dass Strahlung ungehindert zum Boden passieren kann, **dadurch gekennzeichnet, dass** die Wärme durch Emittieren von Wärmestrahlung von einer Strahlungsquelle bereitgestellt wird, die im Trockenapparat über der Auflage vorhanden ist, um den Körper in Kombination mit Wärmeleitung über das Zurückhalteelement, das an dem eingefrorenen Körper angrenzt, zu erreichen.

10. Verfahren nach einem der vorstehenden Ansprüche, **dadurch gekennzeichnet, dass** die Menge des Zuckers in der wässrigen Zusammensetzung aus der Gruppe ausgewählt wird, die aus den Bereichen 20 bis 55 % w/w, 20 bis 50 % w/w, 20 bis 45 % w/w, 25 bis 45 % w/w, 25 bis 40 % w/w, 25 bis 35 % w/w und 27 bis 30 % w/w besteht.

11. Verfahren nach einem der vorstehenden Ansprüche, **dadurch gekennzeichnet, dass** der Zucker monomere und/oder dimere Moleküle umfasst.

12. Verfahren nach Anspruch 11, **dadurch gekennzeichnet, dass** der Glucose, Galactose, Maltose, Sucrose, Trehalose, Fructose, Lactose, Saccharose, Mannit, Sorbit und/oder Xylitol umfasst.

13. Verfahren nach einem der vorstehenden Ansprüche, **dadurch gekennzeichnet, dass** eine Restfeuchte im getrockneten Körper kleiner als 2 % w/w ist.

14. Arzneimittel in der Form eines im Wesentlichen homogenen gefriergetrockneten Körpers mit einem Volumen zwischen 50 und 1000 µl, das ein biologisch aktives Protein umfasst, wobei das Protein in einer festen Matrix eines nichtpolymeren Zuckers dispergiert ist, **dadurch gekennzeichnet, dass** der Körper zwischen ungefähr 21 und 72 % w/v des Zuckers umfasst.

15. Produkt in der Form eines im Wesentlichen homogenen gefriergetrockneten Körpers mit einem Volumen zwischen 50 und 1000 µl, das ein biologisch aktives Protein umfasst, wobei der Körper über ein Verfahren nach einem der Ansprüche 1 bis 13 erlangbar ist.

## Revendications

1. Procédé de production d'un produit médicinal, comprenant une protéine biologiquement active, comprenant les étapes de :
- la fourniture d'une composition aqueuse comprenant un solvant, la protéine biologiquement active et entre 20 % en poids et 60 % en poids d'un sucre non polymérique,
- la congélation de la composition, formant ainsi au moins un corps congelé comprenant le solvant sous la forme congelée,
- la mise du corps congelé dans un appareil de séchage alors qu'il est porté sur un support, le support comprenant un ou plusieurs éléments de retenue qui définissent une ou plusieurs limites du support, où au plus 30 % de la surface du corps est contiguë avec un ou plusieurs éléments de retenue,
- la réduction de la pression dans l'appareil de séchage en dessous de la pression atmosphérique,
- la fourniture de chaleur au corps afin de sublimer le solvant congelé du corps et obtenir un corps séché, le corps lyophilisé ayant un volume entre 50 et 1000 µl.

2. Procédé selon la revendication 1, **caractérisé en ce que** le solvant congelé est sublimé en moins de 48 heures.

3. Procédé selon la revendication 2, **caractérisé en ce que** le solvant congelé est sublimé en moins de 36 heures.

4. Procédé selon la revendication 3, **caractérisé en ce que** le solvant congelé est sublimé en 16 à 24 heures.

5. Procédé selon l'une quelconque des revendications précédentes, **caractérisé en ce qu'**au plus 20 % de la surface du corps est contiguë avec un ou plusieurs éléments de retenue du support.

6. Procédé selon la revendication 5, **caractérisé en ce qu'**au plus 10 % de la surface du corps est contiguë avec un ou plusieurs éléments de retenue du support.

7. Procédé selon l'une quelconque des revendications précédentes, **caractérisé en ce que** le corps congelé est un sphéroïde.

8. Procédé selon l'une quelconque des revendications précédentes, dans lequel l'élément de retenue du support et un sol, le corps étant fourni allongé sur ce sol, et le support est ouvert pour permettre à une radiation de traverser librement vers ledit sol, **caractérisé en ce qu'**au moins une partie de la chaleur est fournie par l'émission d'une radiation thermique à partir d'une source de radiation présente dans l'appareil de séchage au-dessus du support, pour atteindre le corps congelé.

9. Procédé selon l'une quelconque des revendications 1 à 7, dans lequel l'élément de retenue du support est un sol, le corps étant fourni allongé sur ce sol, et le support est ouvert pour permettre à une radiation de traverser librement vers ledit sol, **caractérisé en ce que** la chaleur est fournie par l'émission d'une radiation thermique à partir d'une source de radiation présente dans l'appareil de séchage au-dessus du support, pour atteindre le corps en combinaison avec une conduction de chaleur par l'intermédiaire de l'élément de retenue qui est contigu avec le corps congelé.

10. Procédé selon l'une quelconque des revendications précédentes, **caractérisé en ce que** la quantité de sucre dans la composition aqueuse est choisie dans le groupe constitué des plages de 20 à 55 % en poids, 20 à 50 % en poids, 20 à 45 % en poids, 25 à 45 % en poids, 25 à 40 % en poids, 25 à 35 % en poids et 27 à 30 % en poids.

11. Procédé selon l'une quelconque des revendications précédentes, **caractérisé en ce que** le sucre comprend des molécules monomériques et/ou dimériques.

12. Procédé selon la revendication 11, **caractérisé en ce que** le sucre comprend du glucose, du galactose, du maltose, du sucrose, du tréhalose, du fructose, du lactose, du saccharose, du mannitol, du sorbitol et/ou du xylitol.

13. Procédé selon l'une quelconque des revendications précédentes, **caractérisé en ce qu'**une teneur en humidité résiduelle dans le corps séché est inférieure à 2 % en poids.

14. Produit médicinal sous la forme d'un corps lyophilisé substantiellement homogène ayant un volume entre 50 et 1000 µl comprenant une protéine biologiquement active, la protéine étant dispersée dans une matrice solide d'un sucre non polymérique, **caractérisé en ce que** le corps comprend entre environ 21 et 72 % en poids dudit sucre.

15. Produit sous la forme d'un corps lyophilisé substantiellement homogène ayant un volume entre 50 et 1000 µl, comprenant une protéine biologiquement active, le corps pouvant être obtenu par le biais d'un procédé selon l'une quelconque des revendications 1 à 13.
